(19) Europäisches Patentamt
European Patent Office
Office européen des brevets

(11) EP 4 081 180 B1

(12) **EUROPEAN PATENT SPECIFICATION**

(45) Date of publication and mention of the grant of the patent:
**10.04.2024 Bulletin 2024/15**

(21) Application number: **21707253.7**

(22) Date of filing: **23.02.2021**

(51) International Patent Classification (IPC):
***A61K 8/37*** *(2006.01)* ***A61K 8/31*** *(2006.01)*
***A61K 8/41*** *(2006.01)* ***A61K 8/60*** *(2006.01)*
***A61K 8/73*** *(2006.01)* ***A61K 8/81*** *(2006.01)*
***A61Q 5/04*** *(2006.01)*

(52) Cooperative Patent Classification (CPC):
**A61K 8/37; A61K 8/31; A61K 8/41; A61K 8/604; A61K 8/73; A61K 8/8117; A61K 8/8147; A61Q 5/04**

(86) International application number:
**PCT/EP2021/054414**

(87) International publication number:
**WO 2021/170566 (02.09.2021 Gazette 2021/35)**

(54) **RESHAPING COMPOSITION FOR KERATIN FIBERS**

UMFORMUNGSZUSAMMENSETZUNG FÜR KERATINFASERN

COMPOSITION DE REMISE EN FORME POUR LES FIBRES KÉRATINIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2020 EP 20159951**

(43) Date of publication of application:
**02.11.2022 Bulletin 2022/44**

(73) Proprietor: **Kao Germany GmbH**
**64297 Darmstadt (DE)**

(72) Inventors:
- **BREAKSPEAR, Steven**
**64297 DARMSTADT (DE)**
- **JIAN, Niu**
**64297 DARMSTADT (DE)**
- **NÖCKER, Bernd**
**64297 DARMSTADT (DE)**

(74) Representative: **Miller, Tobias**
**Kao Germany GmbH**
**Pfungstädter Straße 98-100**
**64297 Darmstadt (DE)**

(56) References cited:
**WO-A1-2016/098870**
**WO-A1-2019/074128**
**WO-A2-2007/013006**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 081 180 B1

## Description

### Field of the invention

The present invention is directed to a reshaping composition for keratin fibers, a reshaping process, and kit-of-parts, strictly as defined in the appended claims.

### Background of the invention

Current permanent reshaping processes require steps of reducing the hair and later oxidizing the hair. The reducing step employs reducing agents, such as thioglycolic acid, in order to cleave disulphide bonds within the hair and to allow protein chain movement. In a subsequent step, the bonds are reformed by addition of an oxidizing composition, typically comprising hydrogen peroxide. In Western countries, these well-known processes include cold perming and hot perming techniques, whereas, especially in Asian countries, a digital perming as a specialty of the hot perming process is preferred. For a digital perming process the temperature of the perm is controlled by an electronic device, often being equipped with a microprocessor. These multi-step processes are time-consuming (up to 3 hours for a cold perm/5 hours for a digital perm), require a high level of winding skill from the stylist, cause hair damage and, at worst, can lead to hair loss due to over-processing. Over-processing typically stems from either leaving the reducing agent for too long on the hair, or heating the hair to inappropriate temperatures for a too long time. To avoid over-processing, a test curler is typically carried out on a streak of the client's hair prior to the full process being performed, in order to determine the optimum processing time. This adds further to the time needed and requires further skills/education of the stylist. Apart from all the process challenges, the reducing composition has a strong and acrid odor disliked by stylists and consumers alike. For easing the application of reshaping compositions, WO2016/098870 and WO2019/074128 disclose alkaline non-reducing, non-oxidizing compositions comprising fatty compounds and water-soluble thickening polymers. WO2007/013006 discloses hair styling compositions comprising fatty compounds and a low amount of alkalizing agent.

### Summary of the invention

The first object of the present invention is A non-reducing, non-oxidizing reshaping composition for keratin fibers, preferably human keratin fibers, more preferably human hair, having a pH in the range of 7 to 12, and comprising:

a) one or more alkalizing agent(s), preferably selected from

- ammonia and/or ammonium salt(s), and/or
- organic amine(s) and/or salt(s) of organic amines according to the following general structure:

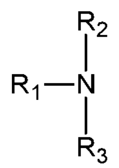

wherein $R_1$, $R_2$, and $R_3$ are independently selected from H, linear $C_1$-$C_6$ alkyl which may be substituted with one hydroxyl group, or branched $C_3$-$C_{12}$ alkyl or alkanol, wherein at least one of $R_1$, $R_2$, or $R_3$ is different from H, and/or

- mixtures of the alkalizing agents from above,

b) one or more lipophilic compound(s) being liquid at 25°C and atmospheric pressure, at a total concentration in the range of 10% to 80% by weight, calculated to the total weight of the composition,

c) one or more surfactant(s),

d) one or more thickening agent(s) being soluble in or mixable with the compounds according to b),

wherein the one or more compound according to d) is one or more oil-soluble thickening polymer and/or hydrogenated vegetable oil, and/or their mixtures,

wherein the total concentration of compound(s) according to a) is in the range of 0.25% to 15% by weight, calculated to the total weight of the composition.

The second object of the present invention is a process for reshaping keratin fibers, preferably human keratin fibers, more preferably human hair comprising the steps of:

i) putting keratin fibers under mechanical tension,
ii) applying to keratin fibers the composition as defined above,
iii) optionally covering keratin fibers with a moisture barrier,
iv) heating the keratin fibers to a temperature in the range of 50°C to 230°C,
v) optionally removing the moisture barrier from keratin fibers,
vi) releasing tension from keratin fibers,
vii) optionally rinsing-off the keratin fibers,

wherein process steps i), ii), and vi), vii) can be executed in either order.

The third object of the present invention is a kit-of-parts comprising the composition as defined above and one or more digital perm curler(s).

**Detailed description of the invention**

Inventors of the present invention have unexpectedly found out that alkaline non-reducing, non-oxidizing compositions comprising an alkalizing agent lipophilic compound, surfactant, and thickening agent, as defined in claim 1, improve curling efficiency during perming, improve cosmetic safety by preventing dripping at process temperatures above 80°C, and improve the cosmetic properties of keratin fibers such as feel, touch, and shine. Moreover, the composition has less to no odor and may be applied without having to cover the keratin fibers with a moisture barrier.

**Reshaping composition**

The present invention is directed to a non-reducing, non-oxidizing reshaping composition for keratin fibers, preferably human keratin fibers, more preferably human hair, having a pH in the range of 7 to 12, and comprising:

a) one or more alkalizing agent(s), preferably selected from

- ammonia and/or ammonium salt(s), and/or
- organic amine(s) and/or salt(s) of organic amines according to the following general structure:

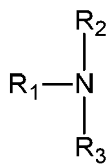

  wherein $R_1$, $R_2$, and $R_3$ are independently selected from H, linear $C_1$-$C_6$ alkyl which may be substituted with one hydroxyl group, or branched $C_3$-$C_{12}$ alkyl or alkanol, wherein at least one of $R_1$, $R_2$, or $R_3$ is different from H, and/or
- mixtures of the alkalizing agents from above,

b) one or more lipophilic compound(s) being liquid at 25°C and atmospheric pressure, at a total concentration in the range of 10% to 80% by weight, calculated to the total weight of the composition,

c) one or more surfactant(s),

d) one or more thickening agent(s) being soluble in or mixable with the compounds according to b),

wherein the one or more compound according to d) is one or more oil-soluble thickening polymer and/or hydrogenated vegetable oil, and/or their mixtures,

wherein the total concentration of compound(s) according to a) is in the range of 0.25% to 15% by weight, calculated to the total weight of the composition.

The term 'non-reducing' within the meaning of the present invention is to be understood that the composition from above is free of agents causing a reduction of disulfide bonds in the keratin fibers. This is usually the case at concentrations of reducing agents below 1% by weight, preferably below 0.1% by weight, calculated to the total weight of the composition. Thus, the term 'non-reducing' does not exclude low amounts of reducing agents which may be necessary in the composition for stabilizing purposes. Most preferably, the composition of the present invention is free of reducing agents.

The term 'non-oxidizing' within the meaning of the present invention is to be understood that the composition from above is free of oxidizing agents causing an oxidation of disulfide bonds in the keratin fibers. This is usually the case at concentrations of oxidizing agents below 1% by weight, preferably below 0.1% by weight, calculated to the total weight of the composition. The term does not exclude low amounts of oxidizing agents, which may be necessary in the composition for stabilizing purposes. Most preferably, the composition of the present invention is free of oxidizing agents.

It is preferred from the viewpoint of keratin fiber reshaping performance that the pH of the composition is 7.5 or more, more preferably 8 or more, further more preferably 8.5 or more.

It is preferred from the viewpoint of keratin fiber reshaping performance, cosmetic safety, and damage to keratin fibers that the pH of the composition is 11 or less, more preferably 10.5 or less, further more preferably 10.0 or less.

For attaining the above-mentioned effects, it is preferred that the pH of the composition is in the range of 7.5 to 11, more preferably 8 to 10.5, further more preferably 8.5 to 10.0.

It is preferred from the viewpoint of rinsability that the composition of the present invention is an aqueous composition. In this respect, it is preferred that the composition of the present invention comprises water at 15% by weight or more, more preferably at 20% by weight or more, further more preferably at 30% by weight or more, still further more preferably at 40% by weight or more, still further more preferably at 50% by weight or more, still further more preferably at 60% by weight or more, still further more preferably at 80% by weight or more, calculated to the total weight of the composition.

It is preferred from the viewpoint of keratin fibers reshaping performance that the composition of the present invention comprises water at 90% by weight or less, more preferably at 85% by weight or less, further more preferably at 75% by weight or less, calculated to the total weight of the composition.

For attaining the above-mentioned effects, it is preferred that the composition of the present invention comprises water in the range of 15% to 90% by weight, more preferably 20% to 85% by weight, further more preferably 30% to 75% by weight, still further more preferably 40% to 75% by weight, still further more preferably 50% to 75% by weight, still further more preferably 60% to 75% by weight, calculated to the total weight of the composition.

The composition of the present invention preferably is an emulsion. Depending on the concentration of compound(s) according to b), it may be presented in the form of an oil-in-water emulsion. However, at higher concentrations of compound(s) according to b), it may also be possible to formulate the composition as an inverse emulsion, i.e. a water-in-oil emulsion.

The composition of the present invention preferably is a permanent waving composition. The composition is particularly beneficial for reshaping with heat, preferably with heat above 50°C, more preferably with heat at 80°C or above.

**Alkalizing agent(s) according to a)**

In principle, any alkalizing agent delivering the desired pH for the composition of the present invention is suitable.

It is preferred from the viewpoint of keratin fiber damage that one or more alkalizing agent(s), preferably selected from

- ammonia and/or ammonium salt(s), and/or
- organic amine(s) and/or salt(s) of organic amines according to the following general structure:

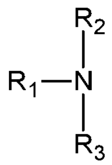

wherein $R_1$, $R_2$, and $R_3$ are independently selected from H, linear $C_1$-$C_6$ alkyl which may be substituted with one hydroxyl group, or branched $C_3$-$C_{12}$ alkyl or alkanol, wherein at least one of $R_1$, $R_2$, or $R_3$ is different from H, and/or

- mixtures of the alkalizing agents from above.

Suitable ammonium salts are inorganic ammonium and/or organic ammonium salts, and/or their mixtures. Inorganic ammonium salts are preferably selected from ammonium chloride, ammonium sulfate, ammonium carbonate, ammonium hydrogen carbonate, ammonium phosphates, ammonium hydrogen phosphates, ammonium dihydrogen phosphates, ammonium nitrate, ammonium bromide, ammonium iodide, ammonium thiosulfate, ammonium molybdate, ammonium vanadate, and/or their mixtures.

Suitable organic ammonium salts are preferably selected from ammonium carbamate, ammonium sulfamate, ammonium citrate, ammonium salicylate, ammonium valerate, ammonium tartarate, ammonium benzoate, ammonium acetate, ammonium formiate, and ammonium lactate, ammonium salts of polymers, and/or their mixtures.

Preferably, from the viewpoint of buffering, the weight ratio of ammonia to ammonium salt(s) in the composition of the present invention is in the range of 10 to 1.

The organic amine(s) and/or salt(s) of organic amines according to the structure above may be selected from mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine, triethylamine, tris-(hydroxymethyl)-aminomethane, and/or aminomethyl propanol. Equally suitable are salts of alkyl and/or alkanol amines with a counterion preferably selected from chloride and/or hydrogen chloride, nitrate, sulphate, phosphate, hydrogen phosphate, dihydrogenphosphate, citrate, acetate, sulphite, benzoate, salicylate.

It is preferred from the viewpoint of keratin fibers reshaping performance and keratin fiber damage that the alkalizing agent according to a) is selected from aminomethyl propanol and/or monoethanolamine, and/or diethanolamine and/or tris-(hydroxymethyl)-aminomethane, and/or ammonia, and/or their salts, and/or their mixtures.

The most preferred alkalizing agent according to a) of the composition of the present invention is tris-(hydroxymethyl)-aminomethane.

The total concentration of compound(s) according to a) is in the range of 0.25% to 15% by weight, calculated to the total weight of the composition.

It is preferred from the viewpoint of providing alkalinity and keratin fibers reshaping performance that the total concentration of alkalizing agent(s) in the composition of the present invention is 0.5% by weight or more further more preferably 1 % by weight or more, calculated to the total weight of the composition.

It is preferred from the viewpoint of keratin fibers reshaping performance and cosmetic safety that the total concentration of alkalizing agent(s) in the composition of the present invention is 10% by weight or less further more preferably 8% by weight or less, calculated to the total weight of the composition.

For attaining the above-mentioned effects, it is preferred that the total concentration of alkalizing agent(s) of the composition of the present invention is in the range of in the range of 0.5% to 10% by weight, more preferably in the range of 1 %to 8% by weight, calculated to the total weight of the composition.

**Lipophilic compounds according to b)**

The composition of the present invention comprises one or more lipophilic compound(s) being liquid at 25°C and atmospheric pressure, at a total concentration in the range of 10% to 80% by weight, calculated to the total weight of the composition, as compound(s) according to b).

It is preferred from the viewpoint of stability that the compound(s) according to b) are selected from natural and/or vegetable oils, mineral oil, and fatty acid esters consisting of linear or branched, saturated or unsaturated fatty acids with $C_{12}$ to $C_{22}$ being esterified with linear or branched primary alcohols with $C_3$ to $C_{12}$, silicones, lauryl alcohol, and/or their mixtures.

Suitable natural and/or vegetable oils are, for example, olive oil, sunflower oil, rapeseed oil, wheatgerm oil, or almond oil.

Suitable silicones are, for example, linear or cyclic silicones with or without amination, such as dimethicone,

trimethicone, and/or amodimethicone.

The preferred compound(s) according to b) are selected from fatty acid esters consisting of linear or branched, saturated or unsaturated fatty acids with $C_{12}$ to $C_{22}$ being esterified with linear or branched primary alcohols with $C_3$ to $C_{12}$, more preferably they are selected from isopropyl palmitate, octyl palmitate, isocetyl palmitate, octyl stearate, oleyl oleate, myristyl myristate, and/or their mixtures, form the viewpoint of curling efficiency and composition stability. The most preferred compound according to b) is isopropyl myristate, from the viewpoint of keratin fiber reshaping, removability with washing, and composition stability.

It is preferred from the viewpoint of keratin fiber reshaping that the total concentration of compound(s) according to b) is 15% by weight or more, more preferably 20% by weight or more, further more preferably 25% by weight or more, calculated to the total weight of the composition.

It is preferred from the viewpoint of removability with washing and composition stability that the total concentration of compound(s) according to b) is 75% by weight or less, more preferably 70% by weight or less, further more preferably 60% by weight or less, still further more preferably 50% by weight or less, still further more preferably 40% by weight or less, calculated to the total weight of the composition.

For attaining the above-mentioned effect, it is preferred that the total concentration of compound(s) according to b) is in the range of 15% to 75% by weight, preferably 20% to 70% by weight, more preferably 25% to 60% by weight, further more preferably 25% to 50% by weight, still further more preferably 25% to 40% by weight, calculated to the total weight of the composition.

**Compounds according to c)**

The composition of the present invention comprises one or more surfactant(s) as compound according to c).

It is preferred from the viewpoint of emulsion stability that the compound(s) according to c) are selected from anionic, non-ionic, amphoteric and/or zwitterionic, and/or cationic surfactants, and/or their mixtures.

Suitable anionic surfactants are selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures.

Suitable alkyl sulfate or preferably ethoxylated alkyl ether sulfate surfactant or mixtures thereof have an alkyl chain length of $C_{10}$ to $C_{22}$.

Suitable example anionic surfactants are laureth sulfates, coceth sulfate, pareth sulfate, capryleth sulphate, myreth sulfate, oleth sulfate, deceth sulfate, trideceth sulfate, coco sulphate, $C_{10}$-$C_{16}$ alkyl sulphate, $C_{11}$-$C_{15}$ alkyl sulphate, $C_{12}$-$C_{18}$ alkyl sulphate, $C_{12}$-$C_{15}$ alkyl sulphate, $C_{12}$-$C_{16}$ alkyl sulphate, $C_{12}$-$C_{13}$ alkyl sulfate, lauryl sulphate, myrystyl sulphate, palm kernel sulphate, cetearyl sulfate, cetyl sulphate, decyl sulphate, oleyl sulphate, behenyl sulphate and/or their salts. All of the aforementioned anionic surfactants may or may not be ethoxylated at various degrees.

Cations for the surfactants may be selected from sodium, potassium, magnesium and/or ammonium.

Suitable non-ionic surfactants are alkyl polyglycosides, ethoxylated triglycerides, ethoxylated fatty alcohols, ethoxylated fatty acid esters, and/or their mixtures.

Preferred non-ionic surfactants are alkyl polyglycosides according to the general structure:

$$R_{23}O(R_{24}O)_tZ_x$$

Wherein Z denotes a carbohydrate with $C_5$ to $C_6$, $R_{23}$ is an alkyl group with $C_8$ to $C_{18}$, $R_{24}$ is methyl, ethyl or propyl, t ranges from 0 to 10, and x ranges from 1 to 5. Suitable compounds according to this structure are $C_9$-$C_{11}$ alkylpolyglycoside, the structures disclosed in EP-A 70 074, and JP 2015-123019A.

The most preferred compounds according to the structure of above are decyl glucoside, lauryl glucoside, and coco glucoside.

Suitable amphoteric/zwitterionic surfactants are compounds according to the general structure(s)

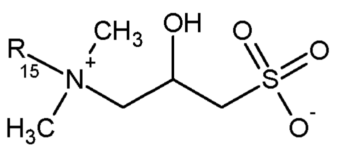

and/or

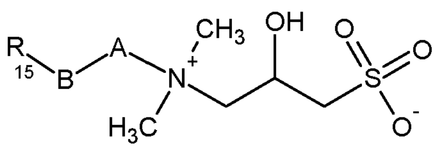

wherein $R_{15}$ is a straight or branched, saturated or unsaturated, substituted or unsubstituted alkyl chain with a carbon number of $C_{10}$ to $C_{22}$, preferably $R_{15}$ is a straight alkyl chain with a carbon number of $C_{10}$ to $C_{16}$, A is a straight alkyl chain with a carbon number of $C_1$ to $C_6$ or a branched alkyl chain with a carbon number of $C_3$ to $C_6$, preferably A is a linear alkyl chain with a carbon number of $C_3$, and B is an amide or an ester group.

Suitable compounds are known as hydroxysultaine surfactants, such as cocoamidopropyl hydroxysultaine, laurylamidopropyl hydroxysultaine, erucamidopropyl hydroxysultaine, lauryl hydroxysultaine, and cocoyl hydroxysultaine, and/or their salt(s).

Further suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

The preferred amphoteric/zwitterionic surfactant(s) is/are selected from alkylamido betaines and/or alkylamidoalkyl betaine surfactants.

Suitable cationic surfactants are of quaternary ammonium structure according to the following general structure

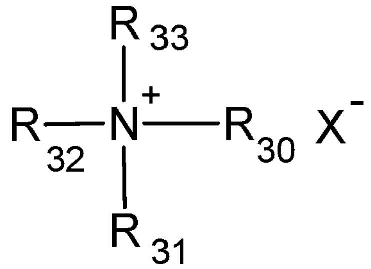

where $R_{30}$ is a saturated or unsaturated, branched or linear alkyl chain with $C_8$-$C_{22}$ or

$R_{34}CONH(CH_2)_n$

where $R_{34}$ is saturated or unsaturated, branched or linear alkyl chain with $C_7$-$C_{21}$ atoms and n has typical value of 1-4 or

$R_{35}COO(CH_2)_n$

where $R_{35}$ is saturated or unsaturated, branched or linear alkyl chain with $C_7$-$C_{21}$ atoms and n has typical value of 1-4, and

$R_{31}$ is unsaturated or saturated, branched or linear alkyl chain with $C_1$-$C_{22}$ atoms or

$R_5CONH(CH_2)_n$

or

$R_6COO(CH_2)_n$

where $R_{34}$, $R_{35}$ and n are same as above.

$R_{32}$ and $R_{33}$ have an alkyl chain with $C_1$ to $C_4$, and $X^-$ typically is chloride, bromide, or methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimethyl ammonium chloride, dioleoylethyl dimethyl ammonium methosulfate, dioleoylethyl hydroxyethylmonium methosulfate, behenyl trimethyl ammonium chloride, and/or their mixtures.

It is preferred from the viewpoint of composition stability that the total concentration of compound(s) according to c) is 0.1% by weight or more, preferably 0.5% by weight or more, further more preferably 0.75% by weight or more, calculated to the total weight of the composition.

It is preferred from the viewpoint of composition stability and cost of goods that the that the total concentration of compound(s) according to c) is 10% by weight or less, further more preferably 7% by weight or less, still further more preferably 5% by weight or less, calculated to the total weight of the composition.

For attaining the above-mentioned effects, it is preferred that the total concentration of compound(s) according to c) is in the range of 0.1% to 10% by weight, preferably 0.5% to 7% by weight, more preferably in the range of 0.75% to 5% by weight, calculated to the total weight of the composition.

**Thickening agent as compounds according to d)**

The composition of the present invention comprises one or more thickening agent(s) being soluble in or mixable with the compounds according to b).

The term 'soluble' within the meaning of the present invention denotes the that the compounds according to d) may be solid at 25°C and atmospheric pressure, but are able to completely dissolve in the compounds according to b). Preferably, the complete dissolution appears at 25°C under atmospheric pressure. However, for the purpose of the present invention, it is sufficient that the thickening agent according to d) dissolves in the compounds according to b) at elevated temperatures leading to sufficient thickening of the composition at these temperatures, for example at process temperatures of keratin fiber treatment at 80°C. Hence, it is not required that at 25°C and atmospheric pressure a complete solution of the compounds according to d) is possible to obtain.

The term 'mixable' denotes that the compounds according to d) may be liquid at 25°C and atmospheric pressure. Hence, the compounds according to d) must be at least partially mixable with the compounds according to b) at 25°C under atmospheric pressure. However, complete mixability must be obtained at process temperatures of keratin fiber treatment at 80°C, then delivering the required thickening effect.

Hence, it is preferred for the purpose of the present invention that the compound(s) according to d) are able to thicken the composition of the present invention at a temperature of 80°C or more. Thus, it is preferred from the viewpoint of heat stability of the composition and keratin fiber reshaping performance that the viscosity of the composition at 80°C is 1,000 mPas or more, more preferably 5,000 mPas or more, further more preferably 10,000 mPas or more, measured by plate-cone viscometry, for example with a Brookfield viscometer with appropriate spindle.

It is preferred from the viewpoint of manufacturing that the compounds according to d) are solid or pasty at 25°C under atmospheric conditions.

The one or more compound according to d) is one or more oil-soluble thickening polymer and/or hydrogenated vegetable oil, and/or their mixtures.

In one aspect of the present invention, the oil-soluble thickening agent according to d) is an oil-soluble thickening polymer, preferably a non-ionic, anionic, non-ionic, and/or cationic oil-soluble thickening polymer, more preferably a non-ionic oil-soluble polymer.

It is preferred from the viewpoint of oil solubility of the thickening polymer that the compound(s) according to d) is a homopolymer or copolymer comprising monomer units of ethylene and/or propylene and/or butylene and/or styrene.

Suitable non-ionic polymers satisfying the description of above are, for example, copolymers of ethylene/butylene with styrene monomers are commercially available under the trade name Kraton polymers, for example Kraton G1701E.

In another aspect of the present invention, the compounds according to d) is/are hydrogenated vegetable oil, which is commercially available under the trade name Dermofeel Viscolid from Evonik Ind. AG.

Thus, preferred thickening agents according to d) are homopolymers or copolymers comprising monomer units of ethylene and/or propylene and/or butylene and/or styrene and hydrogenated vegetable oil, and/or their mixtures.

It is preferred from the viewpoint of thickening effect and reshaping power that the total concentration of compound(s) according to d) is 0.25% by weight or more, more preferably 0.5% by weight or more, further more preferably 0.5% by weight or more, still further more preferably 1% by weight or more, calculated to the total weight of the composition.

It is preferred from the viewpoint of thickening effect, reshaping power, and product safety that the total concentration of compound(s) according to d) is 0.25% by weight or more, more preferably 0.5% by weight or more, further more preferably 1% by weight or more, calculated to the total weight of the composition.

It is preferred from the viewpoint of thickening effect, reshaping power, and rinsability of keratin fibers that the total concentration of compound(s) according to d) is 20% by weight or less, more preferably 15% by weight or less, further more preferably 12% by weight or less, calculated to the total weight of the composition.

For attaining the above-mentioned effects, it is preferred that the total concentration of compound(s) according to d) is in the range of 0.25% to 20% by weight, preferably in the range of 0.5% to 15% by weight, more preferably 1% to 12% by weight, calculated to the total weight of the composition.

Options

iii) optionally covering keratin fibers with a moisture barrier,

iv) heating the keratin fibers to a temperature in the range of 50°C to 230°C,

v) optionally removing the moisture barrier from keratin fibers,

vi) releasing tension from keratin fibers,

vii) optionally rinsing-off the keratin fibers,

wherein process steps i), ii), and vi), vii) can be executed in either order.

It is preferred from the viewpoint of reshaping performance that the process above is a permanent waving process.

It is preferred form the viewpoint of user convenience that for process step i) the keratin fibers are put under mechanical tension on a curler or roller having heating means. After completion of step ii) or optionally step iii), the curler may then be connected to a digital perm machine for heating the curler and the keratin fibers.

It is preferred from the viewpoint of hair reshaping performance that the keratin fibers are heated in step iv) to a temperature of 80°C or more, more preferably to a temperature of 85°C or more, further more preferably to a temperature of 90°C or more.

It is preferred from the viewpoint of minimizing damage that the keratin fibers are heated in step iv) to a temperature of 180°C or less, more preferably to a temperature of 140°C or less, further more preferably to a temperature of 120°C or less.

For attaining the above mentioned effect, it is preferred that the hair is heated in step iv) to a temperature in the range of 80°C to 180°C, more preferably 85°C to 140°C, further more preferably 90°C to 120°C.

It is further preferred from the viewpoint of processing time and speedy hair treatment that the heating time of step iv) is 2 min or more, more preferably 5 min or more, further more preferably 10 min or more, still further more preferably 20 min or more.

It is preferred from the viewpoint of keratin fiber reshaping performance and damage reduction that the heating time of step iv) is 60 min or less, more preferably 45 min or less, still further more preferably 40 min or less.

For attaining the above-mentioned effects, it is preferred that the heating time of step iv) is in the range of 2 min to 60 min, more preferably in the range of 5 min to 45 min, further more preferably in the range of 10 min to 40 min, still further more preferably 20 min to 40 min.

It is preferred from the viewpoint of keratin fiber reshaping performance that in step iii) the keratin fibers are covered with a moisture barrier. The moisture barrier of step iii) may be a foil or wrap impermeable for water vapor, or housing made of a material impermeable for water vapor, with the provision that the selected materials for the moisture barrier are heat resistant up to the selected process temperature.

In principle, many materials are suitable for serving as moisture barrier such as aluminum foil, plastic foil, and/or plastic device, which enclose the curler and/or keratin fiber streak. Alternatively, certain types of anti-flammable fabric is equally suitable. The purpose of the moisture barrier is to keep the keratin fibers moist over the total processing time of heating.

Suitable examples are re-sealable zipper storage bags made of materials such a slow density polyethylene.

For the purpose of the present invention, it is one aspect to cover the keratin fibers with the moisture barrier from the viewpoint of hair reshaping performance. In another aspect from the viewpoint of user convenience, it is preferred that the hair is not covered with a moisture barrier.

## Kit-of-parts

The present invention is also directed to a kit-of-parts comprising the composition as defined above and one or more digital perm curlers(s).

Such kit-of-parts may be offered for sale and have the advantage of user convenience, because it includes composition and devices.

It is further preferred that the kit also comprises one or more moisture barrier for covering the curler as defined in optional step ii) above.

The following examples are to illustrate the invention, but not to limit it.

## EXAMPLES

### Example 1

Compounds according to a) and c) were dissolved in water. Compound according to d) were separately dissolved in compound according to c). The aqueous solution was then heated to 80°C and the oil solution was added under constant stirring. After cooling the mixture, the pH was adjusted and water balance was added.

Human hair streaks (Caucasian, 21 cm long, 2 g per bundle) were purchased from Fischbach + Miller Haar, Laupheim, Germany. The hair streaks were shampooed with a commercially available shampoo under the brand name Goldwell Deep Cleansing Shampoo. Then the streaks were towel dried. Then 1 g of the compositions from above was

applied to the hair streaks with a brush. As a result it was found that the compositions showed strong curling ratios. Moreover, the compositions were easy to apply and remained on the hair streak during processing. The cosmetic feel of the streaks was very good.

The streaks were then wound on perming rods possessing an electrical heating system. The rods were then heated to a temperature in the range of 90°C for 20 min with a digital perming machine. Then the rods were allowed to cool down, and the hair was shampooed with the same shampoo from above. The streaks were then blow-dried.

| Compound | Ingredients | Comparative | Ex 1 | Ex 2 | Ex 3 | Ex 4 | Ex 5 | Ex 6 | Ex 7 | Ex 8 | Ex 9 | Ex 10 | Ex 11 | Ex 12 | Ex 13 |
|---|---|---|---|---|---|---|---|---|---|---|---|---|---|---|---|
| | | % by weight [active matter | | | | | | | | | | | | | |
| a) | Ammonia (25%) | - | 4.0 | - | - | 0.75 | 0.75 | - | - | - | - | - | - | - | - |
| a) | Monoethanolamine | 4.0 | - | 4.0 | - | 0.75 | 0.75 | 4.0 | 4.0 | 4.0 | 4.0 | 4.0 | 4.0 | 4.0 | 8.0 |
| a) | Tris-(hydroxymethyl)-aminomethane | - | - | - | 8.0 | 4.95 | 4.95 | - | - | - | - | - | - | - | - |
| b) | Isopropyl myristate | 25.0 | 25.0 | 25.0 | 25.0 | 25.0 | 25.0 | 25.0 | 25.0 | 10.0 | 60.0 | 25.0 | 25.0 | 25.0 | 25.0 |
| c) | Coco glucoside* | 5.0 | 5.0 | 5.0 | 5.0 | 5.0 | 5.0 | 5.0 | 5.0 | 5.0 | 5.0 | 3.5 | 20.0 | 5.0 | 5.0 |
| d) | Carbopol | 1.0 | - | - | - | - | - | - | - | - | - | - | - | - | - |
| d) | Styrene ethylene/propylene copolymer** | - | 1.0 | 1.0 | 1.0 | 1.0 | - | - | - | - | - | - | - | - | - |
| d) | Dermofeel Viscolid*** | - | - | - | - | - | 1.0 | 0.1 | 1.0 | 1.0 | 1.0 | 1.0 | 1.0 | 8.0 | 1.0 |
| pH | NaOH/HCl | Ad 9.4 | | | | | | | | | | | | | |
| Balance | Water | Ad 100.0 | | | | | | | | | | | | | |

* Plantacare 818 sold by BASF Corp.
** Kraton G1701 E sold by Kraton Polymers LLC
*** Dermofeel Viscolid sold by Evonik Ind. AG

Assessment of curling efficiency was investigated by measuring and calculating the curl ratio L according to the formula:

$$L = (L_0 - L_t)/L_0$$

wherein $L_0$ is the length of the hair streak prior to curling and Lt is the length of the hair streak after the curling experiment. The number is reported as percentage and a higher percentage corresponds to higher curling degree.

Dripping was evaluated by observation of the heated hair curlers at 80°C and the starting time, if any, was recorded.

Hair feel and hair bounce were evaluated by a panel of 10 trained experts who were asked to touch the hair after processing and to rate the feel and bounce on a scale of 1 to 4, wherein 4 indicated the strongest effect. The experts were not informed about the treatment formulation of the hair streaks. The mode value of their assessments were reported below.

The table below reports the experimental results:

| Parameter | Comparative | Ex 1 | Ex 2 | Ex 3 | Ex 4 | Ex 5 | Ex 6 | Ex 7 | Ex 8 | Ex 9 | Ex 10 | Ex 11 | Ex 12 | Ex 13 |
|---|---|---|---|---|---|---|---|---|---|---|---|---|---|---|
| Curl ratio | 6.8 | 10.8 | 10.6 | 11.2 | 12.9 | 13.2 | 12.5 | 12.9 | 10.7 | 10.5 | 12.6 | 13.1 | 12.9 | 12.8 |
| Dripping | - | +++ | +++ | +++ | +++ | +++ | +++ | +++ | + | +++ | ++ | +++ | +++ | +++ |
| Hair feel | + | +++ | +++ | +++ | +++ | +++ | +++ | +++ | +++ | ++ | +++ | +++ | +++ | - |
| Curl bounce | + | ++ | ++ | ++ | +++ | +++ | +++ | +++ | ++ | ++ | +++ | +++ | +++ | +++ |

**Technical effects**

| **Parameter** | +++ | ++ | + | - |
|---|---|---|---|---|
| **Dripping** | No dripping | Dripping after 30 min | Dripping after 15 min | Dripping after 5 min |
| **Hai feel** | Smooth, not greasy | Smooth, slightly greasy | Slightly rough, dry | Rough |
| **Curl bounce** | Intense | Strong | Weak | Very weak / no bounce |

The following example are within the scope of the present invention.

**Example 14**

| | **% by weight** |
|---|---|
| Tris-(hydroxymethyl)-aminomethane | 4.0 |
| Isopropyl myristate | 10.0 |
| Mineral oil | 20.0 |
| Coco glucoside | 3.0 |
| Sodium lauryl sulfate | 1.0 |
| Acrylates copolymer | 1.0 |
| Xanthan gum | 0.5 |
| Styrene ethylene/propylene copolymer | 1.0 |
| NaOH/HCl | ad pH 9.5 |
| Water | ad 100.0 |

## Claims

**1.** A non-reducing, non-oxidizing reshaping composition for keratin fibers, preferably human keratin fibers, more preferably human hair, having a pH in the range of 7 to 12, and comprising:

a) one or more alkalizing agent(s), preferably selected from

- ammonia and/or ammonium salt(s), and/or
- organic amine(s) and/or salt(s) of organic amines according to the following general structure:

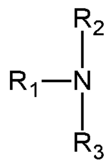

wherein $R_1$, $R_2$, and $R_3$ are independently selected from H, linear $C_1$-$C_6$ alkyl which may be substituted with one hydroxyl group, or branched $C_3$-$C_{12}$ alkyl or alkanol, wherein at least one of $R_1$, $R_2$, or $R_3$ is different from H, and/or
- mixtures of the alkalizing agents from above,

b) one or more lipophilic compound(s) being liquid at 25°C and atmospheric pressure, at a total concentration in the range of 10% to 80% by weight, calculated to the total weight of the composition,
c) one or more surfactant(s),
d) one or more thickening agent(s) being soluble in or mixable with the compounds according to b),

wherein the one or more compound according to d) is one or more oil-soluble thickening polymer and/or hydrogenated vegetable oil, and/or their mixtures, and
wherein the total concentration of compound(s) according to a) is in the range of 0.25% to 15% by weight, calculated to the total weight of the composition.

2. The composition according to claim 1 **characterized in that** the pH is in the range of 7.5 to 11, preferably in the range of 8.0 to 10.5, more preferably in the range of 8.25 to 10, further more preferably in the range of 8.5 to 9.5.

3. The composition according to claims 1 and/or 2 **characterized in that** organic amine(s) as compound(s) according to a) are selected from mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine, tris-(hydroxymethyl)-aminomethane, and/or aminomethyl propanol, and/or their mixtures, preferably they are selected from monoethanolamine, aminomethyl propanol, and/or tris-(hydroxymethyl)-aminomethane, and/or their salt(s), and/or their mixtures.

4. The composition according to any of the preceding claims **characterized in that** the total concentrations of compound(s) according to a) is in the range of 0.25% to 10% by weight, more preferably 0.5% to 8% by weight, calculated to the total weight of the composition.

5. The composition according to any of the preceding claims **characterized in that** the compound(s) according to b) are selected from natural and/or vegetable oils, mineral oil, and fatty acid esters consisting of linear or branched, saturated or unsaturated fatty acids with $C_{12}$ to $C_{22}$ being esterified with linear or branched primary alcohols with $C_3$ to $C_{12}$, and/or silicones, and/or lauryl alcohol, and/or their mixtures, preferably compound(s) according to b) are selected from isopropyl palmitate, octyl palmitate, isocetyl palmitate, octyl stearate, oleyl oleate, myristyl myristate, and/or their mixtures.

6. The composition according to any of the preceding claims **characterized in that** the total concentration of compound(s) according to b) is in the range of 15% to 75% by weight, preferably 20% to 70% by weight, more preferably 25% to 60% by weight, further more preferably 25% to 50% by weight, still further more preferably 25% to 40% by weight, calculated to the total weight of the composition.

7. The composition according to any of the preceding claims **characterized in that** compound(s) according to c) are selected from anionic, non-ionic, amphoteric and/or zwitterionic, and/or cationic surfactants, and/or their mixtures, preferably compound(s) according to c) are selected from non-ionic surfactant(s), preferably from alkyl polyglycoside surfactant(s).

8. The composition according to any of the preceding claims **characterized in that** the total concentration of compound(s) according to c) is in the range of 0.1% to 10% by weight, preferably 0.5% to 7% by weight, more preferably in the range of 0.75% to 5% by weight, calculated to the total weight of the composition.

9. The composition according to any of the preceding claims **characterized in that** the compound(s) according to d) is an oil-soluble thickening polymer, preferably a non-ionic, anionic, non-ionic, and/or cationic oil-soluble thickening polymer, more preferably a non-ionic oil-soluble thickening polymer.

10. The composition according to any of the preceding claims **characterized in that** the compound(s) according to d) is a homopolymer or copolymer comprising monomer units of ethylene and/or propylene and/or butylene and/or styrene, and/or the compound(s) according to d) is/are hydrogenated vegetable oil.

11. The composition according to any of the preceding claims **characterized in that** the total concentration of compound(s) according to d) is in the range of 0.25% to 20% by weight, preferably in the range of 0.5% to 15% by weight, more preferably 1% to 12% by weight, calculated to the total weight of the composition.

12. The composition according to any of the preceding claims **characterized in that** it comprises water in the range of 15% to 85% by weight, more preferably 20% to 85% by weight, further more preferably 30% to 75% by weight, still further more preferably 40% to 75% by weight, still further more preferably 50% to 75% by weight, still further more preferably 60% to 75% by weight, calculated to the total weight of the composition.

13. A process for reshaping keratin fibers, preferably human keratin fibers, more preferably human hair comprising the

steps of:

i) putting keratin fibers under mechanical tension,
ii) applying to keratin fibers the composition as defined in claims 1 to 12,
iii) optionally covering keratin fibers with a moisture barrier,
iv) heating the keratin fibers to a temperature in the range of 50°C to 230°C,
v) optionally removing the moisture barrier from keratin fibers,
vi) releasing tension from keratin fibers,
vii) optionally rinsing-off the keratin fibers,

wherein process steps i), ii), and vi), vii) can be executed in either order.

**14.** The method according to claim 13 **characterized in that** the hair is heated in step iv) to a temperature in the range of 80°C to 180°C, more preferably 85°C to 140°C, further more preferably 90°C to 120°C, and preferably the heating time of step iv) is in the range of 2 min to 60 min, more preferably in the range of 5 min to 45 min, further more preferably in the range of 10 min to 40 min, still further more preferably 20 min to 40 min.

**15.** A kit-of-parts comprising the composition as defined in any of the claims 1 to 12 and one or more digital perm curler(s).

**Patentansprüche**

**1.** Nicht-reduzierende, nicht-oxidierende Umformungszusammensetzung für Keratinfasern, vorzugsweise menschliche Keratinfasern, besonders bevorzugt menschliches Haar, mit einem pH-Wert im Bereich von 7 bis 12, und umfassend:

a) ein oder mehrere Alkalisierungsmittel, vorzugsweise ausgewählt aus

- Ammoniak und/oder Ammoniumsalz(en), und/oder
- organischen Amin(en) und/oder Salz(en) von organischen Aminen gemäß der folgenden allgemeinen Struktur:

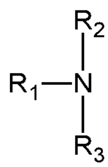

wobei $R_1$, $R_2$ und $R_3$ unabhängig voneinander ausgewählt sind aus H, linearem $C_1$-$C_6$-Alkyl, das mit einer Hydroxylgruppe substituiert sein kann, oder verzweigtem $C_3$-$C_{12}$-Alkyl oder Alkanol, wobei mindestens einer der Reste $R_1$, $R_2$ oder $R_3$ von H verschieden ist, und/oder
- Mischungen der oben genannten Alkalisierungsmittel,

b) eine oder mehrere lipophile Verbindung(en), die bei 25°C und Atmosphärendruck flüssig sind, in einer Gesamtkonzentration im Bereich von 10 bis 80 Gew.-%, berechnet auf das Gesamtgewicht der Zusammensetzung,
c) ein oder mehrere Tensid(e)
d) ein oder mehrere Verdickungsmittel, die in den Verbindungen gemäß b) löslich oder mit diesen mischbar sind,

wobei es sich bei der einen oder mehreren Verbindung(en) gemäß d) um ein oder mehrere öllösliche Verdickungspolymere und/oder hydriertes Pflanzenöl und/oder deren Mischungen handelt.

**2.** Zusammensetzung nach Anspruch 1, **dadurch gekennzeichnet, dass** der pH-Wert im Bereich von 7.5 bis 11, vorzugsweise im Bereich von 8.0 bis 10.5, noch bevorzugter im Bereich von 8.25 bis 10, noch weiter bevorzugt im Bereich von 8.5 bis 9.5 liegt.

**3.** Zusammensetzung nach Anspruch 1 und/oder 2, **dadurch gekennzeichnet, dass** das/die organische(n) Amin(e) als Verbindung(en) nach a) ausgewählt sind aus Mono- und/oder Diethanolamin, Butylethanolamin, Butyldiethanolamin, Dibutylethanolamin, Methylethanolamin, Triethanolamin, N-Lauryldiethanolamin, Diisopropanolamin, Dimethylisopropanolamin, Isopropanolamin, Triisopropanolamin, Isobutanolamin, Tris-(hydroxymethyl)-aminomethan und/oder Aminomethylpropanol und/oder deren Mischungen, vorzugsweise ausgewählt aus Monoethanolamin, Aminomethylpropanol und/oder Tris-(hydroxymethyl)-aminomethan und/oder deren Salz(en) und/oder deren Mischungen.

**4.** Zusammensetzung nach einem der vorhergehenden Ansprüche, **dadurch gekennzeichnet, daß** die Gesamtkonzentration der Verbindung(en) nach a) im Bereich von 0.1 bis 15 Gew.-%, vorzugsweise 0.25 bis 10 Gew.-%, besonders bevorzugt 0.5 bis 8 Gew.-%, bezogen auf das Gesamtgewicht der Zusammensetzung, liegt.

**5.** Zusammensetzung nach einem der vorhergehenden Ansprüche, **dadurch gekennzeichnet, dass** die Verbindung(en) nach b) ausgewählt sind aus natürlichen und/oder pflanzlichen Ölen, Mineralöl und Fettsäureestern, bestehend aus linearen oder verzweigten, gesättigten oder ungesättigten Fettsäuren mit $C_{12}$ bis $C_{22}$, die mit linearen oder verzweigten primären Alkoholen mit $C_3$ bis $C_{12}$ verestert sind, und/oder Silikonen, und/oder Laurylalkohol, und/oder deren Mischungen, vorzugsweise sind die Verbindung(en) gemäß b) ausgewählt aus Isopropylpalmitat, Octylpalmitat, Isocetylpalmitat, Octylstearat, Oleyloleat, Myristylmyristat, und/oder deren Mischungen.

**6.** Zusammensetzung nach einem der vorhergehenden Ansprüche, **dadurch gekennzeichnet, daß** die Gesamtkonzentration der Verbindung(en) nach b) im Bereich von 15 bis 75 Gew.-%, vorzugsweise 20 bis 70 Gew.-%, weiter bevorzugt 25 bis 60 Gew.-%, noch weiter bevorzugt 25 bis 50 Gew.-%, noch weiter bevorzugt 25 bis 40 Gew.-%, bezogen auf das Gesamtgewicht der Zusammensetzung, liegt.

**7.** Zusammensetzung nach einem der vorhergehenden Ansprüche, **dadurch gekennzeichnet, dass** die Verbindung(en) nach c) aus anionischen, nicht-ionischen, amphoteren und/oder zwitterionischen und/oder kationischen Tensiden und/oder deren Mischungen ausgewählt ist/sind, wobei die Verbindung(en) nach c) vorzugsweise aus nicht-ionischen Tensiden, vorzugsweise aus Alkylpolyglycosid-Tensiden ausgewählt ist/sind.

**8.** Zusammensetzung nach einem der vorhergehenden Ansprüche, **dadurch gekennzeichnet, dass** die Gesamtkonzentration der Verbindung(en) gemäß c) im Bereich von 0.1 bis 10 Gew.-%, vorzugsweise 0.5 bis 7 Gew.-%, besonders bevorzugt im Bereich von 0.75 bis 5 Gew.-%, bezogen auf das Gesamtgewicht der Zusammensetzung, liegt.

**9.** Zusammensetzung nach einem der vorangehenden Ansprüche, **dadurch gekennzeichnet, dass** die Verbindung(en) nach d) ein öllösliches Verdickungspolymer, vorzugsweise ein nicht-ionisches, anionisches, nicht-ionisches und/oder kationisches öllösliches Verdickungspolymer, besonders bevorzugt ein nicht-ionisches öllösliches Verdickungspolymer ist.

**10.** Zusammensetzung nach einem der vorhergehenden Ansprüche, **dadurch gekennzeichnet, dass** die Verbindung(en) nach d) ein Homopolymer oder Copolymer ist (sind), das Monomereinheiten von Ethylen und/oder Propylen und/oder Butylen und/oder Styrol umfasst, und/oder die Verbindung(en) gemäß d) ein hydriertes Pflanzenöl ist/sind.

**11.** Zusammensetzung nach einem der vorhergehenden Ansprüche, **dadurch gekennzeichnet, daß** die Gesamtkonzentration der Verbindung(en) nach d) im Bereich von 0.25 bis 20 Gew.-%, vorzugsweise im Bereich von 0.5 bis 15 Gew.-%, besonders bevorzugt 1 bis 12 Gew.-%, bezogen auf das Gesamtgewicht der Zusammensetzung, liegt.

**12.** Zusammensetzung nach einem der vorhergehenden Ansprüche, **dadurch gekennzeichnet, dass** sie Wasser im Bereich von 15 bis 90 Gew.-%, vorzugsweise 20 bis 85 Gew.-%, weiter vorzugsweise 30 bis 75 Gew.-%, noch weiter vorzugsweise 40 bis 75 Gew.-%, noch weiter vorzugsweise 50 bis 75 Gew.-%, noch weiter vorzugsweise 60 bis 75 Gew.-%, bezogen auf das Gesamtgewicht der Zusammensetzung, enthält.

**13.** Verfahren zur Umformung von Keratinfasern, vorzugsweise menschlichen Keratinfasern, besonders bevorzugt menschlichem Haar, umfassend die Schritte

i) mechanische Spannung auf die Keratinfasern ausüben,
ii) Auftragen der in den Ansprüchen 1 bis 12 definierten Zusammensetzung auf Keratinfasern,
iii) gegebenenfalls Abdecken der Keratinfasern mit einer Feuchtigkeitssperre,
iv) Erhitzen der Keratinfasern auf eine Temperatur im Bereich von 50°C bis 230°C,
v) gegebenenfalls Entfernen der Feuchtigkeitsbarriere von den Keratinfasern,
vi) Lösen der Spannung von den Keratinfasern,

vii) optionales Abspülen der Keratinfasern,

wobei die Verfahrensschritte i), ii), und vi), vii) in beliebiger Reihenfolge ausgeführt werden können.

**14.** Verfahren nach Anspruch 13, **dadurch gekennzeichnet, dass** das Haar in Schritt iv) auf eine Temperatur im Bereich von 80°C bis 180°C, bevorzugter 85°C bis 140°C, weiter bevorzugt 90°C bis 120°C, erhitzt wird, und vorzugsweise die Erhitzungszeit von Schritt iv) im Bereich von 2 min bis 60 min, weiter bevorzugt im Bereich von 5 min bis 45 min, noch weiter bevorzugt im Bereich von 10 min bis 40 min, noch weiter bevorzugt 20 min bis 40 min liegt.

**15.** Teilesatz, umfassend die in einem der Ansprüche 1 bis 12 definierte Zusammensetzung und einen oder mehrere digitale Dauerwellenwickler.

## Revendications

**1.** Composition de remise en forme non réductrice et non oxydante pour les fibres kératiniques, de préférence les fibres kératiniques humaines, plus particulièrement les cheveux humains, ayant un pH compris entre 7 et 12, et comprenant:

a) un ou plusieurs agent(s) alcalinisant(s), de préférence choisi(s) parmi

- ammoniaque et/ou sel(s) d'ammonium, et/ou
- une ou plusieurs amines organiques et/ou un ou plusieurs sels d'amines organiques selon la structure générale suivante:

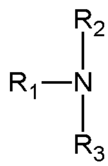

où $R_1$, $R_2$ et $R_3$ sont indépendamment choisis parmi H, alkyle linéaire en $C_1$-$C_6$ pouvant être substitué par un groupe hydroxyle, ou alkyle ramifié en $C_3$-$C_{12}$ ou alcanol, où au moins un des $R_1$, $R_2$ ou $R_3$ est différent de H, et/ou
- des mélanges des agents alcalinisants susmentionnés,

b) un ou plusieurs composés lipophiles liquides à 25°C et à la pression atmosphérique, à une concentration totale comprise entre 10% et 80% en poids, par rapport au poids total de la composition,
c) un ou plusieurs agents tensioactifs,
d) un ou plusieurs agent(s) épaississant(s) soluble(s) ou mélangeable(s) avec les composés selon b),

dans lequel le ou les composés selon d) sont un ou plusieurs polymères épaississants solubles dans l'huile et/ou de l'huile végétale hydrogénée, et/ou leurs mélanges.

**2.** La composition selon la revendication 1 **caractérisée par le fait que** le pH est compris entre 7.5 et 11, de préférence entre 8.0 et 10.5, plus préférentiellement entre 8.25 et 10, encore plus préférentiellement entre 8.5 et 9.5.

**3.** La composition selon les revendications 1 et/ou 2 **caractérisée par le fait que** la ou les amines organiques en tant que composé(s) selon a) sont choisies parmi la mono-et/ou la diéthanolamine, la butyléthanolamine, la butyldiéthanolamine, la dibutyléthanolamine, la méthyléthanolamine, la triéthanolamine, la N-lauryldiéthanolamine, la diisopropanolamine, la diméthyl-isopropanolamine, la diméthyl-isopropanolamine, l'isopropanolamine, l'isopropanolamine, l'isopropanolamine, diméthyl isopropanolamine, isopropanolamine, triisopropanolamine, isobutanolamine, tris-(hydroxyméthyl)-aminométhane, et/ou aminométhyl propanol, et/ou leurs mélanges, de préférence ils sont choisis parmi la monoéthanolamine, l'aminométhyl propanol, et/ou le tris-(hydroxyméthyl)-aminométhane, et/ou leur(s) sel(s), et/ou leurs mélanges.

**4.** La composition selon l'une des revendications précédentes, **caractérisée par le fait que** les concentrations totales

du ou des composés selon a) sont comprises entre 0.1% et 15% en poids, de préférence entre 0.25% et 10% en poids, plus préférentiellement entre 0.5% et 8% en poids, calculées par rapport au poids total de la composition.

**5.** La composition selon l'une quelconque des revendications précédentes **caractérisée en ce que** le(s) composé(s) selon b) sont choisis parmi les huiles naturelles et/ou végétales, les huiles minérales, et les esters d'acides gras constitués d'acides gras linéaires ou ramifiés, saturés ou insaturés en $C_{12}$ à $C_{22}$ estérifiés par des alcools primaires linéaires ou ramifiés en $C_3$ à $C_{12}$, et/ou des silicones, et/ou de l'alcool laurique, et/ou leurs mélanges, de préférence le(s) composé(s) selon b) sont choisis parmi le palmitate d'isopropyle, le palmitate d'octyle, le palmitate d'isocétyle, le stéarate d'octyle, l'oléate d'oléyle, le myristate de myristyle, et/ou leurs mélanges.

**6.** La composition selon l'une quelconque des revendications précédentes, **caractérisée par le fait que** la concentration totale du (des) composé(s) selon b) est comprise entre 15% et 75% en poids, de préférence entre 20% et 70% en poids, de préférence entre 25% et 60% en poids, de préférence encore entre 25% et 50% en poids, de préférence encore entre 25% et 40% en poids, calculée par rapport au poids total de la composition.

**7.** La composition selon l'une quelconque des revendications précédentes **caractérisée en ce que** le(s) composé(s) selon c) sont choisis parmi les tensioactifs anioniques, non ioniques, amphotères et/ou zwitterioniques, et/ou cationiques, et/ou leurs mélanges, de préférence le(s) composé(s) selon c) sont choisis parmi les tensioactifs non ioniques, de préférence parmi les tensioactifs polyglycosides d'alkyle.

**8.** La composition selon l'une des revendications précédentes, **caractérisée par le fait que** la concentration totale du (des) composé(s) selon c) est comprise entre 0.1% et 10% en poids, de préférence entre 0.5% et 7% en poids, plus préférentiellement entre 0.75% et 5% en poids, calculée par rapport au poids total de la composition.

**9.** La composition selon l'une des revendications précédentes **caractérisée par le fait que** le(s) composé(s) selon d) est un polymère épaississant soluble dans l'huile, de préférence un polymère épaississant soluble dans l'huile non ionique, anionique, non ionique et/ou cationique, plus préférentiellement un polymère épaississant soluble dans l'huile non ionique.

**10.** Composition selon l'une quelconque des revendications précédentes, **caractérisée par le fait que** le(s) composé(s) selon d) est un homopolymère ou un copolymère comprenant des unités monomères d'éthylène et/ou de propylène et/ou de butylène et/ou de styrène, et/ou le(s) composé(s) selon d) est/sont une huile végétale hydrogénée.

**11.** La composition selon l'une quelconque des revendications précédentes **caractérisée en ce que** la concentration totale du ou des composés selon d) est comprise entre 0.25% et 20% en poids, de préférence entre 0.5% et 15% en poids, plus préférentiellement entre 1% et 12% en poids, calculée par rapport au poids total de la composition.

**12.** La composition selon l'une des revendications précédentes est **caractérisée par le fait qu'**elle comprend de l'eau dans une fourchette de 15% à 90% en poids, de préférence de 20% à 85% en poids, de préférence de 30% à 75% en poids, de préférence de 40% à 75% en poids, de préférence de 50% à 75% en poids, de préférence de 60% à 75% en poids, calculée par rapport au poids total de la composition.

**13.** Procédé de remodelage des fibres kératiniques, de préférence des fibres kératiniques humaines, et plus particulièrement des cheveux humains, comprenant les étapes suivantes

i) soumettre les fibres kératiniques à une tension mécanique,
ii) appliquer sur les fibres kératiniques la composition telle que définie dans les revendications 1 à 12,
iii) recouvrir éventuellement les fibres kératiniques d'une barrière contre l'humidité,
iv) chauffer les fibres kératiniques à une température comprise entre 50°C et 230°C,
v) éventuellement, retirer la barrière d'humidité des fibres kératiniques,
vi) relâcher la tension des fibres kératiniques,
vii) rincer éventuellement les fibres kératiniques,

les étapes i), ii), vi) et vii) du processus pouvant être exécutées dans n'importe quel ordre.

**14.** Méthode selon la revendication 13, **caractérisée par le fait que** les cheveux sont chauffés à l'étape iv) à une température comprise entre 80°C et 180°C, de préférence entre 85°C et 140°C, de préférence encore entre 90°C et 120°C, et de préférence encore entre 90°C et 120°C, de préférence le temps de chauffage de l'étape iv) est

compris entre 2 min et 60 min, de préférence entre 5 min et 45 min, de préférence encore entre 10 min et 40 min, de préférence encore entre 20 min et 40 min.

**15.** Kit de pièces comprenant la composition telle que définie dans l'une des revendications 1 à 12 et un ou plusieurs bigoudi(s) digital(s).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016098870 A
- WO 2019074128 A
- WO 2007013006 A
- EP 70074 A
- JP 2015123019 A